Europäisches Patentamt

**European Patent Office**

**Office européen des brevets**

(11) Publication number: **0 208 975**
A2

# EUROPEAN PATENT APPLICATION

(21) Application number: **86108810.2**

(22) Date of filing: **28.06.86**

(51) Int. Cl.⁴: **A 61 M 5/315**

(30) Priority: **04.07.85 ES 288140U**
**04.07.85 ES 288141U**
**04.07.85 ES 288142U**

(43) Date of publication of application: **21.01.87**
**Bulletin 87/4**

(84) Designated Contracting States: **AT BE CH DE FR GB IT LI LU NL SE**

(71) Applicant: **Gomez Gomez, Antonio, Moragas 12-22, E-08022 Barcelona (ES)**
Applicant: **Figueras Pradell, Ramon, Dr. Ferrán 11, E-08025 Barcelona (ES)**
Applicant: **Velasco Romera, Martin, Avda. San Antonio Ma Claret, 111-113, E-08025 Barcelona (ES)**

(72) Inventor: **Gomez Gomez, Antonio, Moragas 12-22, E-08022 Barcelona (ES)**
Inventor: **Figueras Pradell, Ramon, Dr. Ferrán 11, E-08025 Barcelona (ES)**
Inventor: **Velasco Romera, Martin, Avda. San Antonio Ma Claret, 111-113, E-08025 Barcelona (ES)**

(74) Representative: **Dipl.-Ing. H. Marsch Dipl.-Ing. K. Sparing Dipl.-Phys.Dr. W.H. Röhl Patentanwälte, Rethelstrasse 123, D-4000 Düsseldorf (DE)**

(54) **Syringe for producing vacuum-controlled suction.**

(57) A syringe for producing controlled vacuum suction for medical purpose, particularly for suctional puncture cytological studies, and optionally standard fluid injection functions like conventional syringes, of the general type comprising a cylinder (1) finishing at the front end in a suction and/or injection tube (2) designed for taking a corresponding needle, and inside the cylinder a plunger (3) which, when it is displaced in the cylinder on the up stroke, produces a vacuum, wherein the cylinder and the plunger stem (5) are provided with supplementary means (6, 8) for mutual locking designed for simple, swift anchoring of this stem in respect of the cylinder so as to maintain a desired vacuum by preventing the plunger to move back to the front end of the cylinder while carrying out vacuum suction, said locking being simply releasable, at the user's discretion, so that the plunger returns to its initial position and cancels the vacuum.

This invention refers to a syringe for producing vacuum-controlled suction for medical purposes, particularly for cytological studies by suctional puncture. It can also perform the normal functions of fluid injection like conventional syringes of the general type made up of a cylinder finishing at the front in a suction and/or injection tube adapted for receiving a corresponding needle, with a plunger acting inside the cylinder which, when displaced on an operational up-stroke in the cylinder, produces a vacuum inside it.

Suctional punctures for cytological studies have hitherto been carried out by diverse techniques. The commonest of these consists of using a very heavy gun in which a standard syringe is inserted to perform the suctional puncture. This system has major drawbacks in that the unit proves very heavy for carrying out the puncture operation and, in addition, it is nesessary to sterilize the gun every time it is used again. This sterilization is a slow tiresome operation and in a hospital or medical centre where many functions have to be performed a large number of guns are needed, and they are, furthermore, very expensive.

In another system that is also used, once aspiration is completed, an additional stop part is applied to the syringe specially adapted for holding the plunger in the suction position, whilst acting on the puncture with the syringe. Although in this case the drawback of the heavy weight of the gun in the previous instance is eliminated, there are two much more serious disadvantages: the former of these is that the stop-piece often gets buried in the plunger or jumps from its lock position, and the plunger is then forced down to the bottom of the cylinder and the vacuum is interrupted suddenly, which proves highly dangerous as the material previously absorbed may be sent back involuntarily. The other disadvantage of this aspiration function system consists in the fact that the stop always has the same length and the magnitude of the vacuum will therefore always be the same, with no possibility of variation. A further disadvantage of this system is that it requires two people to fit the stop.

As opposed to all these drawbacks in the familiar systems, the syringe which is the subject of this invention offers the following advantages:

- it requires no additional device and may be used by one person alone.

- being extremely handy and very light, it is very easy to use, and the exact spot where the suctional puncture is to be carried out can be reached with great ease.

- it maintains the vacuum and is released with absolute safety.

- it enables the magnitude of the vacuum to be set as desired.

- its mouth can accept any type of hyperdermic needle, which reduces the cost of use.

- it can be used both for sucking or aspirating and for injecting.

- its permits the technique of pneumo-oncography, i.e., the percutaneous injection of a quantity of air into a solid mammary lesion for radiodiagnostic purposes and for later aspiration of material for cytological study, without withdrawing the needle from the lesion.

- the syringe is throw-away and, therefore, does not require any sterilization.

The essential distinguishing feature of the syringe is that the cylinder and the plunger stem are fitted with respective supplementary means for mutual anchoring, designed so that this stem may be locked in respect of the cylinder quickly and simply in order to maintain the desired vacuum. This stops the plunger moving back to the front of the cylinder whilst carrying out the vacuum suction, at the same time as it allows this locking to be released simply, at the user's discretion, so that the plunger may return to its initial position, thereby cancelling the vacuum.

Three ways in which the invention may be put into practice are illustrated in the drawings attached, merely by way of non-restrictive example.

Figs. 1, 2 and 3 are respective elevational, sectional and non-sectional, and plan views of one way in which the syringe in question may be made.

Figs. 4, 5 and 6 illustrate similar views to Figs. 1, 2 and 3 of a second way of putting the invention into practice.

Figs. 7, 8 and 9, also similar to Figs. 1, 2 and 3, are views of a third way of putting the invention into practice.

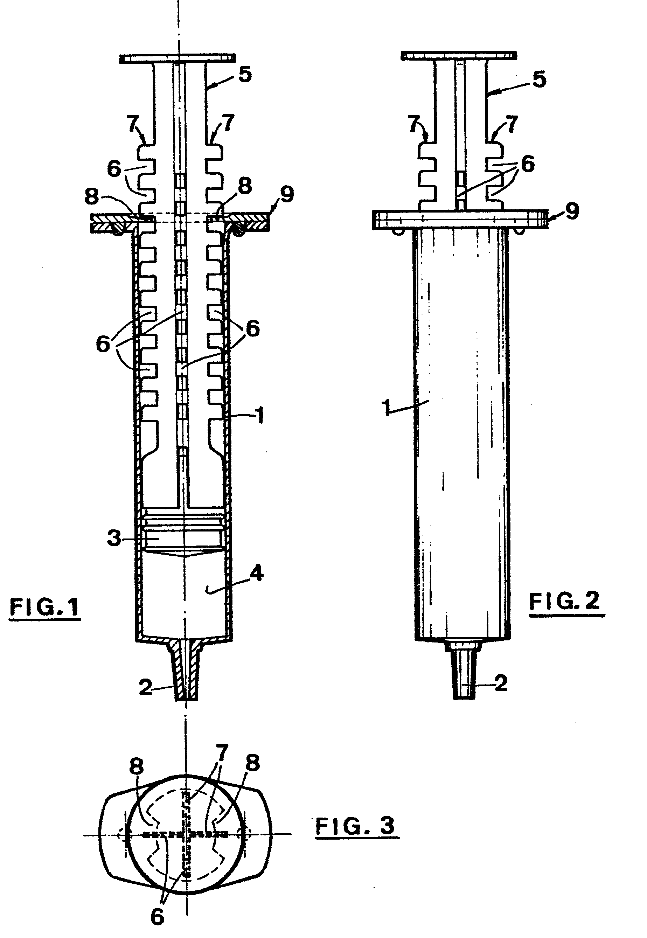

Referring first of all to the form of construction illustrated in Figs. 1, 2 and 3, we may see that the syringe consists of a cylinder 1, finishing conventionally at the front in a tube 2 for suction and/or injection, specially adapted to take a corresponding needle (not shown).

Inside cylinder 1 there is a plunger 3 which, when it is displaced inside the cylinder 1 on the up strocke, produces the vacuum inside the cylinder in chamber 4.

The cylinder 1 and the stem 5 of the plunger 3 are fitted with respective supplementary means for mutual locking which consist of a series of notches 6 cut in the side edge of the stem 5 of the plunger 3.

These notches are aligned in at least one longitudinal lug 7, and four of these lugs 7 are shown in the form of construction illustrated in Figs. 1, 2 and 3.

This arrangement is designed so that a corresponding lip 8, protruding inwards and fitted at the mouth 9 of the cylinder 1, is engaged in one notch 6 of at least one lug by turning the stem 5 in the mouth 9.

In these circumstances, the plunger is anchored in respect of the cylinder 1. It is only then necessary to unscrew the stem 5 in the mouth 9 for the lip 8 to come out of the notch 6 where it is engaged, so that the lock is released and the plunger 3 may then return to its initial position.

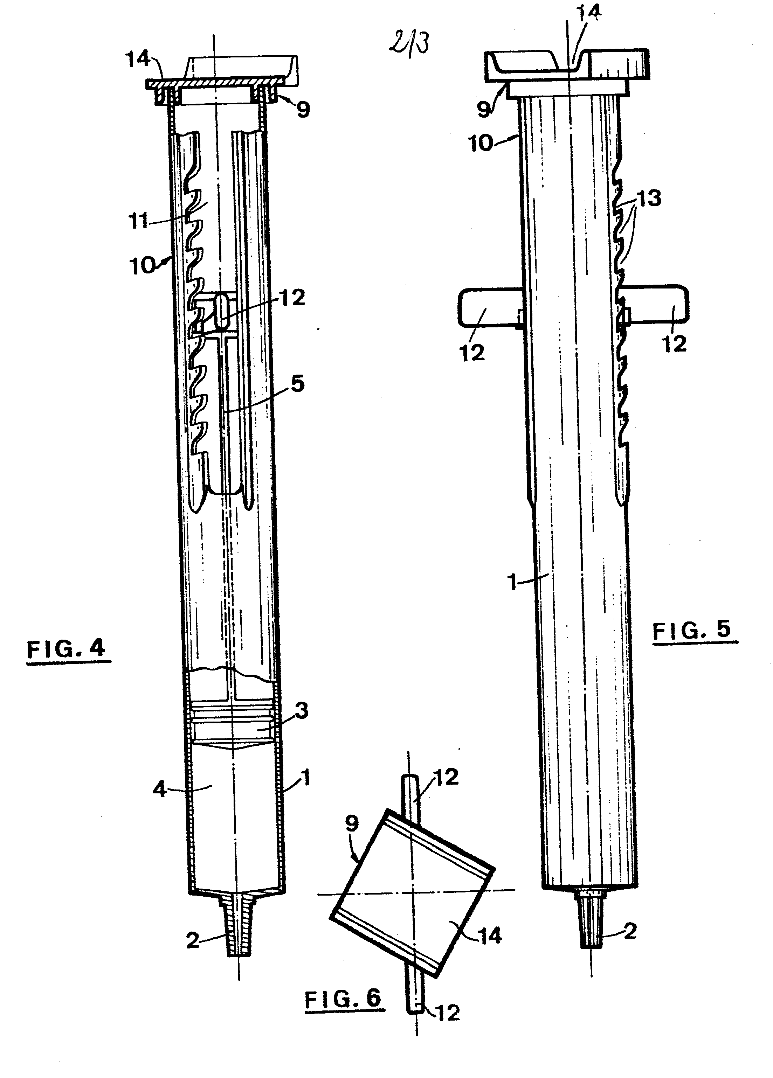

In the second system shown in Figs. 4, 5 and 6, the same components as in Figs. 1, 2 and 3 are affected by the same reference numbers. In this second system the supplementary locking means between the cylinder 1 and the stem 5 of the plunger 3 comprise an extension 10 to the cylinder 1 with two diametrically opposed grooves cut in it, from which two transverse tangs 12, linked at right angles to the stem 5, protrude.

One of the edges of each of these grooves 11 is fitted with a series of notches 13 designed so that, when the stem 5 is turned gently in the cylinder 1, these tangs 12 are engaged in respective notches 13. In this position the plunger 3 is locked in respect of the cylinder 1 and it is only then necessary to turn the stem 5 back in the mouth 9 for this locking to be released, so that the plunger 3 may return to its initial position.

The outside end of this extension 10 to the cylinder 1 is provided with a flat 14 designed to support the user's thumb when carrying out suction, which is when the index and middle fingers pull back the stem 5 by resting them on the above-mentioned transverse tangs 12.

This arrangement enables the syringe to be used comfortably with only one hand since the operative suction stroke takes place through this opposed action of the thumb on the flat 14 and the index and middle fingers on the tangs 12.

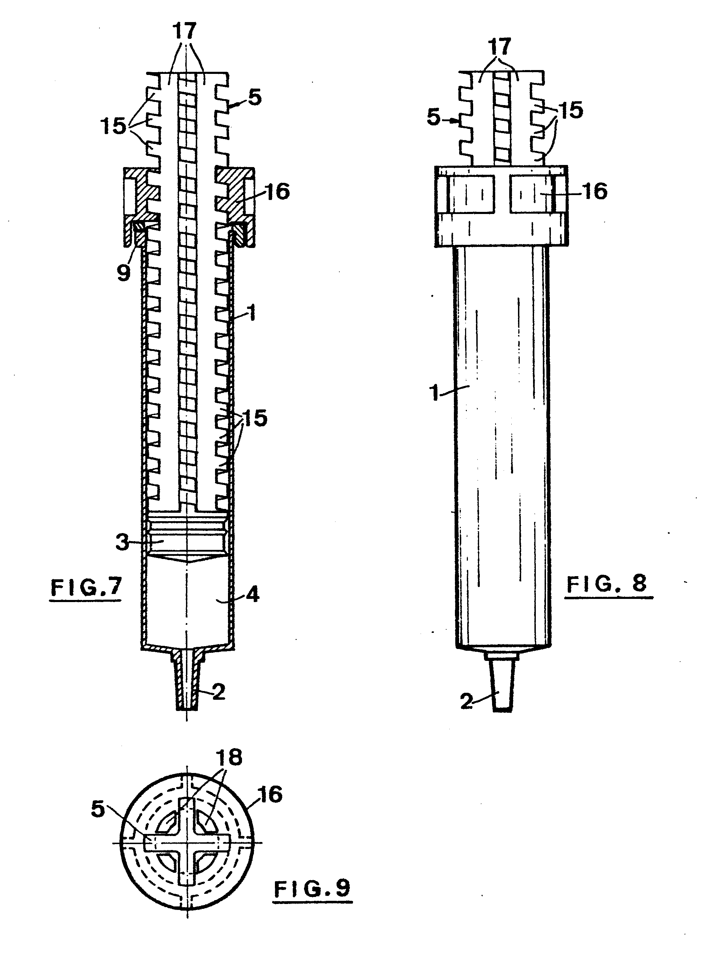

In Figs. 7, 8 and 9, which refer to a third way of putting the invention into practice, the components common to the systems shown in the previous figures, have the same reference numbers. In this third system, the supplementary means of locking the cylinder 1 and the stem 5 consist of threading 15 around the stem 5 designed to work with a nut 16 resting on the outside edge of the mouth 9 of the cylinder 1.

The cylinder 1 and stem 5 assembly is equipped with means designed to stop them turning in respect of each other, so that when this nut 16 is turned the stem 5 can be displaced in relation to the cylinder 1 in either direction, depending on the way in which nut 16 is turned, increasing or reducing the vacuum suction as desired.

The above-mentioned means for stopping the stem 5 from turning in respect of the cylinder 1 consist of at least one lengthwise groove 17, which in the case illustrated in Figs. 7 and 8 are four grooves machined in the stem 5, and corresponding inward-facing lips 18, forming part of the mouth 9 and intended for engaging in these lengthwise grooves 17 in the stem 5.

CLAIMS

1. Syringe for producing controlled vacuum suction for medical purposes, particularly for suctional puncture cytological studies, and optionally standard fluid injection functions like conventional syringes, of the general type comprising a cylinder finishing at the front end in a suction and/or injection tube designed for taking a corresponding needle, and inside the cylinder a plunger which, when it is displaced in the cylinder on the up stroke, produces the vacuum, characterized in that the cylinder and the plunger stem are provided with supplementary means for mutual locking designed for simple, swift anchoring of this stem in respect of the cylinder so as to maintain a desired vacuum by preventing the plunger to move back to the front end of the cylinder while carrying out vacuum suction, said locking being simply releasable, at the user's discretion, so that the plunger returns to its initial position and cancels the vacuum.

2. Syringe according to claim 1, characterized in that the respective supplementary mutual locking means comprises a series of notches cut in the side edge of the plunger stem in alignment with at least one lengthwise lug and designed so that a corresponding inwardly protruding lip in the mouth of the cylinder engages in a notch of at least one lug by turning the stem in respect of the mouth, the plunger being locked in this position relative to the cylinder, the stem being releasable upon returning it into its non-engaging previous position.

3. Syringe according to claim 1, characterized in that the respective ancilliary mutual locking means consist of an extension to the cylinder in which at least two diametrically opposed lengthwise grooves are out, from which two transverse tangs, incorporated at right angles into the stem, protrude, one of the edges of each of these grooves being provided with a series of notches designed so that when the stem is turned gently in the cylinder these tangs engage in respective notches for anchoring the plunger in respect of the cylinder, the stem being releasable upon returning it into its non-engaging previous position.

4. Syringe according to claim 3, characterized in that there is a flat on the outside end of the cylinder extension designed to support the user's thumb when carrying out suction, whereupon the index and middle fingers rest on the afore-mentioned transverse tangs to pull back the stem.

5. Syringe according to claim 1, characterized in that the respective ancilliary mutual locking means comprises threads round the stem designed to cooperate with a nut resting on the outside edge of the cylinder mouth and means to stop the stem turning in respect of the cylinder, so that when this nut is turned the stem is displaced in relation to the cylinder in one direction or the other, depending on which way the nut is turned, thereby increasing or reducing the vacuum suction as desired.

6. Syringe according to claim 5, characterized in that the afore-mentioned means for stopping the stem from turning in respect of the cylinder consist of at least one lengthwise groove cut in the edge of the stem and of at least one corresponding inward-facing lip, forming part of the cylinder and designed to engage in the above-mentioned lengthwise groove in the stem.

1/3    0208975

FIG.1

FIG.2

FIG. 3

FIG. 4

FIG. 5

FIG. 6

3/3

0208975

FIG.7

FIG. 8

FIG.9